# EUROPEAN PATENT APPLICATION

(11) **EP 4 751 716 A1**
(43) Date of publication of application: **03.06.2026**
(21) Application number: 25219263.8
(22) Date of filing: 28.11.2025
(51) Int. Cl.: A61K 9/10, A61K 35/39, A61K 47/42, A61P 3/10

(54) **FORMULATION OF HUMAN PLURIPOTENT STEM CELL-DERIVED ISLETS AND PROCESS FOR PREPARING THE SAME**

(30) Priority: 29.11.2024 CN 202411745069
(71) Applicant: Hangzhou Reprogenix Bioscience, Inc., Hangzhou, Zhejiang 311100 (CN)
(72) Inventor: WU, Shuangshuang, ZHEJIANG, 311100 (CN); LIANG, Ting, ZHEJIANG, 311100 (CN); HUANG, Jie, ZHEJIANG, 311100 (CN); CAO, Nan, ZHEJIANG, 311100 (CN)
(74) Representative: Cabinet Becker et Associés

(57) **Abstract**

The present disclosure provides a formulation of human pluripotent stem cell-derived islets, which is capable of maintaining the structure and biological activity of the human pluripotent stem cell-derived islets during transportation; and said formulation could be used in the manufacture of injections, the final product of injections meets the requirements including sterility, apyrogenicity, safety and stability.

## Description

### Technical Field

The present invention relates to the field of stem cells. Specifically, the present invention provides a formulation of human pluripotent stem cell-derived islets and process for preparing the same, useful in the treatment of insulin deficiency-related diseases through hepatic portal vein or under rectus sheath injection.

### Bacground

Islet beta cell (BC) transplantation may offer a definitive cure for Type I diabetes. However, the limited availability of donor beta cells restricts the application of this treatment as a clinical therapy. Pluripotent stem (PS) cells are capable of unlimited reproduction and differentiation into various types of cells. Therefore, PS cells are a promising source of beta cells. Human pluripotent stem cell-derived islets are produced by directionally inducing human pluripotent stem cells to differentiate into cell aggregates (cell spheroids) with a diameter greater than 50 µm. Currently, there are no reports on formulating human pluripotent stem cell-derived islets into a formulation. For stem cell-derived islets, after directed differentiation in a cell factory, they need to be transported to medical institutions for transplantation of human pluripotent stem cell-derived islets, and face problems of decreased cell viability during the transportation.

### Summary

The formulation of human pluripotent stem cell-derived islets provided by the present invention is suitable for long-distance transportation, can maintain the structure and biological activity of the human pluripotent stem cell-derived islets during long-distance transportation, and can be used to prepare an injection; it is particularly suitable for treating insulin deficiency-related diseases through hepatic portal vein or under rectus sheath injection after long-distance transportation. For an injuction prepared from said formulation, the final product of injections meets the requirements including sterility, apyrogenicity, safety and stability.

The present invention, by providing a formulation of human pluripotent stem cell-derived islets, maintains the proportion of NKX6.1+ C-PEP+ cells and/or a high level of biological activity in human pluripotent stem cell-derived islets in the formulation.

In a first aspect, the present invention provides a formulation of human pluripotent stem cell-derived islets.

In some embodiments, a formulation of human pluripotent stem cell-derived islets comprises pluripotent stem cell (hPSC)-derived islets, human serum albumin, and a basal medium, wherein the mass/volume concentration (m/v) of human serum albumin is 0%-5% (i.e., 0-5 g/100 mL). The applicant found that, for added human serum albumin, a concentration of 0% leads to more severe cell adhesion, and a human serum albumin concentration higher than 5% results in significantly decreased biological activity of the preserved human pluripotent stem cell-derived islets.

In some embodiments, the basal medium in the formulation of human pluripotent stem cell-derived islets is selected from the group consisting of 0.9% physiological saline, water for injection, 5% glucose, or a mixed medium thereof, preferably water for injection. The inventors found that when the basal medium is selected from the group consisting of phosphate buffered saline, cell morphology and activity decrease significantly.

In some embodiments, the human pluripotent stem cell-derived islets content per milliliter of the formulation of human pluripotent stem cell-derived islets is 10-20 million single cells, preferably 4-12 million single cells. The applicant found that a concentration of human pluripotent stem cell-derived islets content at 1 million single cells does not affect cell viability and morphology, but requies a large volume of formulation liquid, which is uneconomical and inconvenient for concentration operations prior to clinical use.

In some embodiments, pluripotent stem cell (hPSC)-derived islet aggregates in per milliliter formulation of human pluripotent stem cell-derived islets equivalent to 1-20 million single cells derived from human pluripotent stem cell-derived islets. Preferably, pluripotent stem cell (hPSC)-derived islet aggregates in per milliliter formulation equivalent to 2-16 million single cells derived from human pluripotent stem cell-derived islets.

In some embodiments, in per milliliter formulation of human pluripotent stem cell-derived islets, the human pluripotent stem cell-derived islets content is 1,000-10,000 IEQ, preferably 2,000-8,000 IEQ, more preferably 2,500-7,000 IEQ. The applicant found that a human pluripotent stem cell-derived islets content higher than 9000 IEQ leads to poor cell activity and morphology. One IEQ of human pluripotent stem cell-derived islets is equivalent to a single cell amount of 1,000-2,000.

In some embodiments, the mass/volume concentration (m/v) of human serum albumin is 0%-5% (namely, 0-5 g/100 mL), and is specifically selected from the group consisting of 0%, 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.05%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.05%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.05%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4.0%, 4.05%, 4.1%, 4.15%, 4.2%, 4.25%, 4.3%, 4.35%, 4.4%, 4.45%, 4.5%, 4.55%, 4.6%, 4.65%, 4.7%, 4.75%, 4.8%, 4.85%, 4.9%, 4.95%, or 5%.

In some embodiments, the formulation of human pluripotent stem cell-derived islets further comprises an osmotic pressure regulator and a pH regulator.

In some embodiments, the osmotic pressure regulator is selected from the group consisting of: sodium chloride, potassium chloride, calcium chloride, magnesium chloride, zinc sulfate, glucose, trehalose, or a combination thereof. In some embodiments, preferably, the osmotic pressure regulator is a combination of sodium chloride, potassium chloride, and calcium chloride; or a combination of sodium chloride, potassium chloride, calcium chloride, and magnesium chloride; or a combination of sodium chloride, potassium chloride, calcium chloride, and glucose.

In some embodiments, the formulation of human pluripotent stem cell-derived islets further comprises a pH regulator, which controls the pH of the formulation in the range of 6.5-8.0.

In some embodiments, the formulation of human pluripotent stem cell-derived islets further comprises an antioxidant or an additional stabilizer, or a combination thereof. The additional stabilizer is selected from one or more of histidine, sodium gluconate, pyruvate (sodium pyruvate), Nicotinamide ITS (1X), linoleic acid, and Vitamin E.

In some embodiments, the formulation of human pluripotent stem cell-derived islets comprises:
pluripotent stem cell (hPSC)-derived islets in the form of cell aggregates of 50-350 µm, wherein pluripotent stem cell (hPSC)-derived islet aggregates in per milliliter formulation equivalent to 1-20 million single cells derived from human pluripotent stem cell-derived islets; or the density of pluripotent stem cell (hPSC)-derived islet aggregates is 1,000-10,000 IEQ;
0%-5% human serum albumin;
an osmotic pressure regulator, which is selected from a combination of sodium chloride, potassium chloride, and calcium chloride, or the combination with further addition of magnesium chloride;
a pH regulator, which is selected from sodium lactate, or a combination of sodium bicarbonate, sodium citrate, and citric acid;
optionally, an additional stabilizer, which is selected from histidine, sodium gluconate, or a combination thereof; and
optionally, an antioxidant, which is selected from one or more of pyruvate and Nicotinamide.

In some embodiments, the formulation of human pluripotent stem cell-derived islets comprises:
pluripotent stem cell (hPSC)-derived islets in the form of cell aggregates of 50-350 µm, wherein pluripotent stem cell (hPSC)-derived islet aggregates in per milliliter formulation equivalent to 2-16 million single cells derived from human pluripotent stem cell-derived islets; or the density of pluripotent stem cell (hPSC)-derived islet aggregates is 2,000-8,000 IEQ;
0%-5% human serum albumin;
an osmotic pressure regulator, which is selected from 1.0-9.0 mg/ml sodium chloride, 0.1-3.0 mg/ml potassium chloride, 0.01-2.0 mg/ml calcium chloride, and 0-1.0 mg/ml magnesium chloride;
preferably a combination of 6.0 mg/ml sodium chloride, 0.3 mg/ml potassium chloride and 0.2 mg/ml calcium chloride, or a combination of 6.0 mg/ml sodium chloride, 0.3 mg/ml potassium chloride, 0.2 mg/ml calcium chloride and 0.3 mg/ml magnesium chloride;
a pH regulator, which is selected from 1.5-5.0 mg/ml sodium lactate, 20-30 mM HEPES, or a combination of 2.35 mg/ml sodium bicarbonate, 0.2 mg/ml sodium citrate and 0.14 mg/ml citric acid; preferably 3.1 mg/ml sodium lactate, 25 mM HEPES, or a combination of 2.35 mg/ml sodium bicarbonate, 0.2 mg/ml sodium citrate and 0.14 mg/ml citric acid;
optionally, an additional stabilizer, which is selected from 0-6.0 mg/ml histidine; and
optionally, an antioxidant, which is selected from 0-10 mM Nicotinamide.

In some embodiments, the amount of the osmotic pressure regulator sodium chloride is selected from 1.0-9.0 mg/ml, the specific values of which include 1.0, 2.0, 3.0, 4.0, 5.0, 6.0, 7.0, 8.0, and 9.0 mg/ml. In some embodiments, the amount of the osmotic pressure regulator potassium chloride is selected from 0.1-3.0 mg/ml, the specific values of which include 0.1, 0.5, 1.0, 1.5, 2.0, 2.5, and 3.0 mg/ml. In some embodiments, the amount of the osmotic pressure regulator calcium chloride is selected from 0.01-2.0 mg/ml, the specific values of which include 0.01, 0.025, 0.05, 0.075, 0.1, 0.25, 0.5, 0.75, 1.0, 1.25, 1.5, 1.75, and 2.0 mg/ml. In some embodiments, the amount of the osmotic pressure regulator magnesium chloride is selected from 0-1.0 mg/ml, the specific values of which include 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, and 1.0 mg/ml.

In some embodiments, the amount of the pH regulator sodium lactate is 1.5-5.0 mg/ml, the specific values of which include 1.5, 1.55, 1.6, 1.65, 1.7, 1.75, 1.8, 1.85, 1.9, 1.95, 2.0, 2.05, 2.1, 2.15, 2.2, 2.25, 2.3, 2.35, 2.4, 2.45, 2.5, 2.55, 2.6, 2.65, 2.7, 2.75, 2.8, 2.85, 2.9, 2.95, 3.0, 3.05, 3.1, 3.15, 3.2, 3.25, 3.3, 3.35, 3.4, 3.45, 3.5, 3.55, 3.6, 3.65, 3.7, 3.75, 3.8, 3.85, 3.9, 3.95, 4.0, 4.05, 4.1, 4.15, 4.2, 4.25, 4.3, 4.35, 4.4, 4.45, 4.5, 4.55, 4.6, 4.65, 4.7, 4.75, 4.8, 4.85, 4.9, 4.95, and 5 mg/ml.

In some embodiments, the amount of histidine is 0-6.0 mg/ml; preferably 0-2.0 mg/ml. Specific values are selected from 0, 0.05, 0.1, 0.15, 0.2, 0.25, 0.3, 0.35, 0.4, 0.45, 0.5, 0.55, 0.6, 0.65, 0.7, 0.75, 0.8, 0.85, 0.9, 0.95, 1.0, 1.05, 1.1, 1.15, 1.2, 1.25, 1.3, 1.35, 1.4, 1.45, 1.5, 1.55, 1.6, 1.65, 1.7, 1.75, 1.8, 1.85, 1.9, 1.95, and 2.0.

In some embodiments, the formulation of human pluripotent stem cell-derived islets comprises:
pluripotent stem cell (hPSC)-derived islets in the form of cell aggregates of 50-350 µm,
pluripotent stem cell (hPSC)-derived islet aggregates in per milliliter formulation equivalent to 2-16 million single cells derived from human pluripotent stem cell-derived islets; or the density of pluripotent stem cell (hPSC)-derived islet aggregates is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
3.1 mg/ml sodium lactate;
0-6.0 mg/ml, preferably 0-2.0 mg/ml, of histidine; and
0-10 mM Nicotinamide.

In some embodiments, the formulation of human pluripotent stem cell-derived islets comprises:
pluripotent stem cell (hPSC)-derived islets in the form of cell aggregates of 50-350 µm, wherein pluripotent stem cell (hPSC)-derived islet aggregates in per milliliter formulation equivalent to 2-16 million single cells derived from human pluripotent stem cell-derived islets; or the density of pluripotent stem cell (hPSC)-derived islet aggregates is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
25 mM HEPES or a combination of 25 mM HEPES and 3.1 mg/ml sodium lactate;
0-2.0 mg/ml histidine; and
0-10 mM Nicotinamide.

In some embodiments, the formulation of human pluripotent stem cell-derived islets comprises:
pluripotent stem cell (hPSC)-derived islets in the form of cell aggregates of 50-350 µm, wherein pluripotent stem cell (hPSC)-derived islet aggregates in per milliliter formulation equivalent to 2-16 million single cells derived from human pluripotent stem cell-derived islets; or the density of pluripotent stem cell (hPSC)-derived islet aggregates is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
a combination of 2.35 mg/ml sodium bicarbonate, 0.2 mg/ml sodium citrate and 0.14 mg/ml citric acid;
optionally, an additional stabilizer, which is selected from 0-2.0 mg/ml histidine;
optionally, an antioxidant, which is selected from 0-10 mM Nicotinamide.

In some embodiments, the formulation of human pluripotent stem cell-derived islets comprises:
pluripotent stem cell (hPSC)-derived islets in the form of cell aggregates of 50-350 µm, wherein pluripotent stem cell (hPSC)-derived islet aggregates in per milliliter formulation equivalent to 2-16 million single cells derived from human pluripotent stem cell-derived islets content of; or the density of pluripotent stem cell (hPSC)-derived islet aggregates is 2,000-8,000 IEQ;
0%-5% human serum albumin;
9.0 mg/ml sodium chloride (equivalent to 0.9% physiological saline);
optionally, an additional stabilizer, which is selected from 0-2.0 mg/ml histidine;
optionally, an antioxidant, which is selected from 0-10 mM Nicotinamide.

In some embodiments, the human pluripotent stem cell-derived islets express one or more of PDX1, NKX6.1, C-PEP, ISL1, ARX, GCG, SST, HHEX, MAFA, VMAT1, and CHGA. In some embodiments, the human pluripotent stem cell-derived islets express both NKX6.1 and C-PEP.

In some embodiments, the human pluripotent stem cell-derived islets are differentiated from human pluripotent stem cells. The human pluripotent stem cells are selected from the group consisting of: non-gene-edited or optionally gene-edited human embryonic stem cells, human induced pluripotent stem cells (hiPSCs), or combinations thereof.

In some embodiments, the human induced pluripotent stem cells (hiPSCs) are derived from human adipose tissue, derived from human pancreas, derived from human epidermis, derived from blood, or derived from urine, etc. The gene editing is selected from the group consisting of CRISPR/Cas technology, engineered zinc finger nuclease (ZFN) technology, transcription activator-like effector (TALE) technology, or TALE-CRISPR/Cas technology. Preferably, the CRISPR/Cas technology is CRISPR-Cas3, CRISPR-Cas9, CRISPR-Cas12, CRISPR-Cas13, CRISPR-CasX, or CRISPR-IscB system.

In some embodiments, the human pluripotent stem cell-derived islets in the formulation of human pluripotent stem cell-derived islets are NKX6.1+ and C-PEP+ double-positive. Preferably, after the formulation is stored at 4°C to 28°C (±3°C) for 24 hours, at least 20% of human pluripotent stem cell-derived islets are NKX6.1+ and C-PEP+ double-positive; preferably, at least 30% of human pluripotent stem cell-derived islets are NKX6.1+ and C-PEP+ double-positive, at least 35% of human pluripotent stem cell-derived islets are NKX6.1+ and C-PEP+ double-positive, at least 40% of human pluripotent stem cell-derived islets are NKX6.1+ and C-PEP+ double-positive, at least 45% of human pluripotent stem cell-derived islets are NKX6.1+ and C-PEP+ double-positive, at least 50% of human pluripotent stem cell-derived islets are NKX6.1+ and C-PEP+ double-positive, at least 55% of human pluripotent stem cell-derived islets are NKX6.1+ and C-PEP+ double-positive. Specific data can be selected from any integer or decimal value from at least 20% to 95%. More preferably, after the formulation is stored at 16°C to 22°C (±3°C) for 24 hours, at least 20% of human pluripotent stem cell-derived islets are NKX6.1+ and C-PEP+ double-positive; preferably, at least 30% of human pluripotent stem cell-derived islets are NKX6.1+ and C-PEP+ double-positive, at least 35% of human pluripotent stem cell-derived islets are NKX6.1+ and C-PEP+ double-positive, at least 40% of human pluripotent stem cell-derived islets are NKX6.1+ and C-PEP+ double-positive, at least 45% of human pluripotent stem cell-derived islets are NKX6.1+ and C-PEP+ double-positive, at least 50% of human pluripotent stem cell-derived islets are NKX6.1+ and C-PEP+ double-positive, at least 55% of human pluripotent stem cell-derived islets are NKX6.1+ and C-PEP+ double-positive. Specific data can be selected from any integer or decimal value from at least 20% to 95%.

In some embodiments, the pluripotent stem cell-derived islets in the formulation of human pluripotent stem cell-derived islets are cell aggregates with a diameter greater than 50 µm; preferably cell aggregates with a diameter greater of 50-350 µm. Specific values are selected from any integer or decimal value from 50 µm to 350 µm, for example, 50 µm, 100 µm, 150 µm, 200 µm, 250 µm, 300 µm, 350 µm. In some embodiments, 95% of the cell aggregates in the formulation of human pluripotent stem cell-derived islets have diameters between 50-350 µm, between 75-300 µm, or between 75-275 µm.

In some embodiments, the formulation of human pluripotent stem cell-derived islets, after storage at 4°C to 28°C (±3°C) for 24 hours, preferably at 4°C to 22°C (±3°C) for 24 hours, has human pluripotent stem cell-derived islets maintaining a cell viability (biological activity) above 85%, preferably maintaining a cell viability above 85%, 90%, 92.5%, 95%, 97.5%, or 98%.

In some embodiments, the formulation of human pluripotent stem cell-derived islets, after storage at 4°C to 28°C (±3°C) for 24 hours, preferably at 4°C to 22°C (±3°C) for 24 hours, maintains good cell morphology. Said good cell morphology is manifested as smooth cell aggregates surfaces, uniform size, and good refractivity. In some embodiments, the formulation of human pluripotent stem cell-derived islets, after storage at 4°C to 28°C (±3°C) for 24 hours, the islet score of the cell aggregates is between 6.0 and 10. Specific islet scores of the cell aggregates are selected from the group consisting of 6.0, 6.5, 7.0, 7.5, 8.0, 8.5, 9.0, 9.5, and 10.

In some embodiments, the formulation of human pluripotent stem cell-derived islets, after storage at 4°C to 22°C (±3°C) for 24 hours, maintains a good proportion of NKX6.1-positive and C-PEP-positive double-positive cells, good biological activity, and good cell aggregates morphology.

In some embodiments, the temperatures in the present invention, including 4°C to 37°C, 4°C to 28°C, 4°C to 22°C, and 16°C to 22°C, include any integer or one-decimal-place value within these ranges.

In some embodiments, the formulation of human pluripotent stem cell-derived islets is an injection. In some embodiments, the injection is an injection for hepatic portal vein or an injection for under rectus sheath, more preferably, an injection for under rectus sheath.

In another aspect, the present invention provides a process for preparing the formulation of human pluripotent stem cell-derived islets according to any one of the preceding embodiments, comprising the steps of:
(1) cell preservation composition formulation: adding human serum albumin and other components to a basal medium, and mixing uniformly to prepare a cell preservation composition for human pluripotent stem cell-derived islets; and
(2) mixing the cell preservation composition with human pluripotent stem cell-derived islets, and filling into packaging containers.

In another aspect, the present invention provides use of the formulation of human pluripotent stem cell-derived islets according to any one of the preceding embodiments in the manufacture of a medicament for the treatment of insulin deficiency. In some embodiments, preferably, the medicament is an injection for hepatic portal vein or an injection for under rectus sheath, more preferably, an injection for sub-anterior rectus sheath.

In another aspect, the present invention provides the formulation of human pluripotent stem cell-derived islets according to any one of the preceding embodiments for use in the treatment of insulin deficiency. In some embodiments, preferably, the formulation is an injection for hepatic portal vein or an injection for under rectus sheath, more preferably, an injection for sub-anterior rectus sheath.

In another aspect, the present invention provides use of a cell preservation composition for human pluripotent stem cell-derived islets in long-distance transportation, wherein the preservation composition consists of the components in the formulation according to any one of the preceding embodiments without human pluripotent stem cell-derived islets.

In some embodiments, preferably, the long-distance transportation temperature is 4°C, 10°C, 16°C, 22°C, or 28°C. In some embodiments, preferably, the transportation time does not exceed 96 hours, 72 hours, 48 hours, 36 hours, 24 hours, 20 hours, 16 hours, 12 hours, 8 hours, 6 hours, or 4 hours.

The process for differentiating and preparing the human pluripotent stem cell-derived islets in the present invention may be referred to WO2023/097513A1 and PCT/CN2023/096328. The human pluripotent stem cell-derived islets in the present invention also include the islet aggregates obtained after treatment using the resuscitation culture medium and/or suspension culture medium described in CN118726236A. The disclosures of WO2023/097513A1, PCT/CN2023/096328, and CN118726236A, particularly those concerning the process for differentiating and preparing human pluripotent stem cell-derived islets and the process for culturing resuscitative islet aggregates, are incorporated herein by reference in their entirety.

### Definitions

In this application, the use of the singular includes the plural unless specifically stated otherwise. It must be noted that, as used in the specification, the singular forms "a," "an" and "the" include plural referents unless the context clearly dictates otherwise.

Reference in the specification to "some embodiments," "an embodiment," "one embodiment" or "other embodiments" means that a particular feature, structure, or characteristic described in connection with the embodiments is included in at least some embodiments, but not necessarily all embodiments, of the present disclosures.

As used in this specification and claim(s), the words "comprising" (and any form of comprising, such as "having," "including" or "containing") are inclusive or open-ended and do not exclude additional, unrecited elements or method steps. It is contemplated that any embodiment discussed in this specification can be implemented with respect to any method or composition of the present disclosure, and vice versa. Furthermore, compositions of the present disclosure can be used to achieve methods of the present disclosure. In some embodiments, the words "having," "including" or "containing" may be replaced by close-ended expressions, such as "consists of" or "consisting of."

As used herein, the term "about" and its grammatical equivalents, when referring to a numerical value, may include that specific value itself and a range of values from 10% less or more than the value.

As used herein, the term "about" or its grammatical equivalents in relation to a reference numerical value and its grammatical equivalents as used herein can include the numerical value itself and a range of values plus or minus 10% from that numerical value. Where specific values are described in this application and the claims, it should be assumed, unless stated otherwise, that the specific value includes an acceptable error margin. In the absence of a specific definition, the acceptable error margin includes a range of values from the numerical value by ±10%, 9%, 8%, 7%, 6%, 5%, 4%, 3%, 2%, or 1%.

As used herein, the term "optionally" includes the situations of "selecting" or "not selecting", "using" or "not using". For example, optionally an additional stabilizer includes both the situation of "using an additional stabilizer" and "not using an additional stabilizer".

As used herein, the mass/volume concentration (m/v) of human serum albumin has the unit g/100 mL and is interchangeable with %. For example: a mass/volume concentration (m/v) of human serum albumin of 0%-0.5% is equivalent to 0-0.5 g/100 mL; a mass/volume concentration of human serum albumin of 20% is equivalent to 20 g/100 mL; and a mass/volume concentration of human serum albumin of 0.25% is equivalent to 0.25 g/100 mL.

As used herein, the terms "diabetes", "insulin deficiency", and their grammatical equivalents may refer to diseases characterized by prolonged high blood glucose levels. For example, as used herein, the term "diabetes" and its grammatical equivalents may refer to all or any types of diabetes, including but not limited to, type 1 diabetes, type 2 diabetes, prediabetes, cystic fibrosis-related diabetes, surgically-induced diabetes, gestational diabetes, mitochondrial diabetes, a risk of any combination thereof, for example, a need for islet transplantation into a subject in need of such treatment. In some cases, the diabetes may be in the form of monogenic diabetes.

Unless otherwise specified, the terms "islet", "islets", "islet equivalent", "islet-like cells", "pancreaticislet" may refer to hormone-producing cells present in the pancreas of an organism. In embodiments, islets can be distinguished from pancreatic progenitor or precursor cells. Islets may include different cell types, including but not limited to, pancreatic α cells, pancreatic β cells, pancreatic δ cells, pancreatic F cells, and/or pancreatic ε cells. Islets may also refer to a group of cells, a cell cluster, etc.

The term "stem cell" as used herein, can refer to an undifferentiated cell which is capable of proliferation and giving rise to more progenitor cells having the ability to generate a large number of mother cells that can in turn give rise to differentiated, or differentiable daughter cells. The daughter cells themselves can be induced to proliferate and produce progeny that subsequently differentiate into one or more mature cell types, while also retaining one or more cells with parental developmental potential. Formally, it is possible that cells that begin as stem cells might proceed toward a differentiated phenotype, but then "reverse" and re-express the stem cell phenotype, a term often referred to as "dedifferentiation" or "reprogramming" or "retro-differentiation" by persons of ordinary skill in the art. As used herein, the term "pluripotent stem cell" includes embryonic stem cells, induced pluripotent stem cells, placental stem cells, etc.

The term "pluripotent" as used herein can refer to a cell with the capacity, under different conditions, to differentiate to more than one differentiated cell type, and preferably to differentiate to cell types characteristic of all three germ cell layers. Pluripotent cells are characterized primarily by their ability to differentiate to more than one cell type, preferably to all three germ layers, using, for example, a nude mouse teratoma formation assay. Pluripotency is also evidenced by the expression of embryonic stem (ES) cell markers, although the preferred test for pluripotency is the demonstration of the capacity to differentiate into cells of each of the three germ layers. It should be noted that simply culturing such cells does not, on its own, render them pluripotent. Reprogrammed pluripotent cells (e.g. iPS cells as that term is defined herein) also have the characteristic of the capacity of extended passaging without loss of growth potential, relative to primary cell parents, which generally have capacity for only a limited number of divisions in culture.

The terms "human pluripotent stem cell-derived islets", "hPSC-islets" or "hPSC-islets" refer to hormone-producing cells similar to those in the pancreas of an organism, obtained through human pluripotent stem cell differentiation and have mature insulin secretion function. Flow cytometry analysis shows that hPSC-islets contain on average about 30-60% pancreatic β cells, 5-20% pancreatic α cells, and 1-10% pancreatic δ cells.

The terms "stem cell-derived β cell," "SC-β cell," "functional β cell," "functional pancreatic β cell," "mature SC-β cell," and their grammatical equivalents can refer to cells (e.g., non-native pancreatic β cells) that display at least one marker indicative of a pancreatic β cell (e.g., NKX6.1, C-PEP or PDX-1), expresses insulin, and display a glucose stimulated insulin secretion (GSIS) response characteristic of an endogenous mature β cell. In some embodiments, the terms "SC-β cell" and "non-native β cell" as used herein are interchangeable. In some embodiments, the "SC-β cell" comprises a mature pancreatic cell. It is to be understood that the SC-β cells need not be derived (e.g., directly) from stem cells, as the methods of the disclosure are capable of deriving SC-β cells from any insulin-positive endocrine cell or precursor thereof using any cell as a starting point (e.g., one can use embryonic stem cells, induced-pluripotent stem cells, progenitor cells, partially reprogrammed somatic cells (e.g., a somatic cell which has been partially reprogrammed to an intermediate state between an induced pluripotent stem cell and the somatic cell from which it was derived), multipotent cells, totipotent cells, a trans differentiated version of any of the foregoing cells.

NKX6.1 & C-PEP double-positive cell population (%) of islet aggregates refers to the proportion of the NKX6.1 and C-PEP double-positive cell population in flow cytometry detection, performed after digesting the islet aggregates into single cells.

By "under rectus sheath transplantation", it refers to introduce the transplants or grafts into a position beneath and within the rectus sheath, preferably into the space between the rectus sheath and musculus rectus abdominis. By "sub-anterior rectus sheath transplantation", it specifically refers to administering the transplants or grafts into a position under the front side of the rectus sheath, preferably into the space between the anterior rectus sheath and the front side of the rectus abdominis and reaching a position under the anterior rectus sheath, as shown in WO2023/227068A1 and its drawings.

The term "rectus abdominis", "abdominis rectus", "musculus rectus abdominis" or "abdominal muscle" can be used interchangeably in the context of the present invention, which refer to the two parallel flat muscles on either side of the linea alba and extending along the whole length of front of the abdomen.

The term "islet equivalent" or "IEQ" is a standardized measure to describe the islet mass to be transplanted. Reference can be made to e.g. Lembert N, et al., for the determination of IEQ (Lembert N, et al. Areal density measurement is a convenient method for the determination of porcine islet equivalents without counting and sizing individual islets. Cell Transplant. 2003;12(1):33-41).

### Beneficial Effects

1. In the screening of stabilizers, the present invention unexpectedly found that human serum albumin, within an appropriate concentration range, can maintain the biological activity of islets and prevent their physical adhesion to culture dishes or packaging containers. Specifically, in some embodiments, for added human serum albumin, a concentration of 0% leads to cell aggregation and more severe adhesion, resulting in significant losses during collection and affecting yield. A human serum albumin concentration higher than 5% results in a biological activity of the preserved human pluripotent stem cell-derived islets below 90%, as well as significant decrease in cell viability, β-cell expression, and islet score. Besides, this application further found that low concentrations of histidine can be used as an auxiliary component, by observing that the addition of low concentration (0-2.0 mg/mL) histidine increases β-cell expression, while the addition of high concentration (6.0 mg/mL) histidine leads to severe cell adhesion as well as decreased cell viability and β-cell expression.
2. In the screening of osmotic pressure regulators, the present invention found that using only physiological saline as the basic solution resulted in poor maintenance of islet aggregates morphology. When using water for injection as the basal medium, sodium chloride, potassium chloride, and calcium chloride are necessary components for maintaining osmotic pressure. If the osmotic pressure is too low, islets exhibit swelling defects. Calcium chloride, at its suitable concentration range of 0.01 mg/ml to 2.0 mg/ml, helps maintain the biological activity of islets; but at its high concentration of 5.0 mg/ml, the osmotic pressure reaches as high as 372 mOsmol/kg, the islets become compact and prone to dehydration, potentially affecting the normal biological activity. Magnesium chloride, as an advantageous component, aids in maintaining the proportion of islet β-cells, cell aggregates morphology, and biological activity. While low concentrations of glucose (0-10 mM) can serve as an auxiliary osmolyte, the addition of a high glucose concentration (27.7 mM) was observed to cause darkening of the cell aggregates, accompanied by a failure to maintain cell viability and functional markers.
3. In the screening of pH regulators, the present invention found that selecting phosphate buffered saline failed to maintain the morphology of islet aggregates. This application also found that sodium lactate is a necessary component for maintaining the pH of the formulation liquid, effectively preserving islets biological activity within the concentration range of 1.5 mg/ml to 5.0 mg/ml. HEPES is a non-essential component that can assist in maintaining the pH of the formulation liquid (7.2-8.0).
4. In the screening of formulation cell density, the present invention found that at a higher formulation density (9,000 IEQ/mL), the viability of the islets, β-cell expression, and islet score all exhibited a marked decrease over 72 hours of storage, accompanied by noticeably worsened cell adhesion. These drawbacks were mitigated upon reduction of the formulation density (below 8,000 IEQ/mL).
5. In the screening for formulation stability, the present invention found that the formulation liquid provided herein can maintain the islet score, cell viability, and NKX6.1/C-PEP levels within a temperature range of 4°C to 28°C.

### Detailed Description

Solutions of the present disclosure will be explained below in connection with the examples. It will be appreciated by those skilled in the art that the following examples are merely illustrative of the present disclosure and should not be construed as limiting the scope of the present disclosure. Techniques or conditions that are not specified in the examples shall be performed in accordance with the techniques or conditions described in the literatures in the art or in accordance with the product instruction. Reagents or instruments without indicating the manufacturer are all common products that are commercially available. For the kits and their applications mentioned in the present invention, the operations are performed by referring to the kit instructions. In the absence of mentioned operation methods, the operations shall follow the instructions of commercially available instruments or the common operation methods of a person skilled in the art.

### Test Method

The morphology and size of the human pluripotent stem cell-derived islets were observed under a microscope at 0-72 hours after formulation preparation, and the diameter size distribution was analyzed using CellView.

Cell viability was detected using the NucleoCounter^{®} NC-200^{™}, specifically: Human pluripotent stem cell-derived islet aggregates were digested into single cells using Accutase, and Via1-Cassette^{™} with pipetted samples was inserted into the instrument slot for detection using NucleoCounter^{®} NC-200^{™} cell counter.

The expression of Markers related to the biological activity of human pluripotent stem cell-derived islets, such as the proportion of β cells (NKX6.1+ and C-PEP+), the proportion of endocrine cells (CHGA+), and the proportion of α cells (ARX+/GCG+), was detected by flow cytometry. Specifically: Human pluripotent stem cell-derived islet aggregates were digested into single cells using Accutase, fixed overnight with BD Cytofix/Cytoperm^{™} solution, permeabilized with Wash Buffer, and then incubated with antibodies. After washing, the cells were filtered through a cell strainer and detected using a flow cytometer.

The islet score was evaluated according to the detailed criteria in Table 1 and calculated by summing the scores across all groups.

**Table 1. Detailed Criteria for Islet aggregates Scores (Maximum Score: 10 points)**

| | |
|---|---|
| Size Distribution | A score of 2 is assigned when the cell aggregates size is between 50 to 350 µm and ≥98% show a uniform size distribution. A score of 1.5 is given when a minimal number (≤5%) are smaller than 50 µm or larger than 350 µm. A score of 1 is assigned if this proportion is >5% but <50%. A score of 0.5 is given if the proportion reaches ≥50%. A score of 0 is assigned when no cell aggregates sizes are within the 50 to 350 µm range. |
| Debris | A score of 2 is assigned for the absence of cell aggregates fragments and very few scattered cells. A score of 1.5 is given for the presence of a minimal number (≤5%) of fragments and a small quantity of scattered cells. A score of 1 is assigned if there is wall-adherence at the edge of the culture dishes, large floccules in the medium, or if fragments and scattered cells account for >5% but <50%. A score of 0.5 is given in cases of severe wall-adherence, a large number of floccules in the medium, or if fragments and scattered cells account for ≥50%. A score of 0 is assigned if floccules or scattered cells account for all. |
| Density | A score of 2 is assigned for compact cell aggregates with clear contours. A score of 1.5 is given for slightly non-compact aggregates with mildly blurred contours. A score of 1 is assigned for moderately compact aggregates with moderately clear contours. A score of 0.5 is given for non-compact aggregates with unclear contours and capacity of no dispersing after pipetting through micropipettes. A score of 0.5 is given for non-compact aggregates with unclear contours and dispersing after pipetting through micropipettes. |
| Morphology | A score of 2 is assigned for regular cell aggregates morphology without fusion or adhesion. A score of 1.5 is given when a minimal number (≤5%) of cell aggregates are irregular or only fusion or adhesion of 2 cell aggregates are observed. A score of 1 is assigned if >5% but <50% of cell aggregates are irregular, or if fusion or adhesion of 2 to 5 cell aggregates that can be dispersed by pipetting through micropipettes are observed. A score of 0.5 is given if ≥50% of cell aggregates are irregular or there is significant fusion or adhesion of cell aggregates. A score of 0 is assigned for completely irregular morphology, and fusion into aggregates larger than 350 µm, or adhesion into sheets. |
| Refractivity | A score of 2 is assigned when ≥ 98% of the cell aggregates population exhibits good refractivity. A score of 1.5 is given when a minimal number (≤ 5%) of aggregates show impaired central refractivity. A score of 1 is assigned if this proportion is >5% but <50%. A score of 0.5 is given if the proportion reaches ≥ 50%. A score of 0 is assigned when all aggregates lack refractivity. |

### Preparation of Cell Formulation Liquid

The formulations of human pluripotent stem cell-derived islets were prepared according to the respective example and comparative formulation compositions.

The human pluripotent stem cell-derived islets used in the Examples of the present invention were all prepared using the method described in Stages 1-6 on pages 36-37 of WO2023/097513A1, with cell aggregates diameters of 50-350 µm.

Using water for injection as the basal medium, components were added according to the respective formulations shown in the tables and mixed uniformly, followed by sterile filtration through a 0.22 µm filter to prepare the human pluripotent stem cell-derived islets preservation solution. Subsequently, the respective cell preservation composition solutions and the human pluripotent stem cell-derived islets were prepared into formulations at a concentration of 1,000-9,000 IEQ/ml, which were filled into formulation bags and stored at 22°C (±3°C). Unless otherwise specified (as in Example 3), the content of human pluripotent stem cell-derived islets per milliliter formulation of human pluripotent stem cell-derived islets was 6,000 IEQ.

### Example 1. Stabilizer Human Serum Albumin Concentration Screening

Formulations of human pluripotent stem cell-derived islets containing 0%, 0.5%, and 5% (% means 50 mg/ml) human serum albumin were prepared according to the compositions in Table 2, for measuring pH value and osmotic pressure (mOsmol/kg), as well as islet score, cell viability (%), and the proportion of cell surface markers NKX6.1+ and C-PEP+ (i.e., efficiency %) at 0H, 24H, 48H, and 72H.

**Table 2. Formulation Screening with Different Concentrations of Stabilizer**

| **Formulation No.** | **Sodium chloride mg/ml** | **Potassium chloride mg/ml** | **Calcium chloride mg/ml** | **Sodium lactate mg/ml** | **HSA %** | **Histidine mg/ml** | **pH** | **Osmolarity mOsmol/kg** |
|---|---|---|---|---|---|---|---|---|
| Comparative Formulation A1 | 6.0 | 0.3 | 0.2 | 3.1 | 0.00 | - | 6.72 | 253 |
| Formulation A2 | 6.0 | 0.3 | 0.2 | 3.1 | 0.50 | - | 6.8 | 275 |
| Comparative Formulation A3 | 6.0 | 0.3 | 0.2 | 3.1 | 5.00 | - | 7.18 | 256 |
| Formulation A4 | 6.0 | 0.3 | 0.2 | 3.1 | - | - | 6.72 | 253 |
| Formulation A5 | 6.0 | 0.3 | 0.2 | 3.1 | - | 2.0 | 7.55 | 265 |
| Comparative Formulation A6 | 6.0 | 0.3 | 0.2 | 3.1 | 0.50 | 6.0 | 7.68 | 290 |

**Table 2-1. Test Results for Different Concentrations of the Stabilizer HSA**

| **Formul** | **0H** | | | **24H** | | | **48H** | | | **72H** | | |
|---|---|---|---|---|---|---|---|---|---|---|---|---|
| **ation No.** | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Comparat ive Formulati on A1 | | | | 9.25 | 93.20 | 64.70 | 8.25 | 94.70 | 66 | 6.25 | 94.20 | 65.40 |
| Formulati on A2 | 10 | 95.70 | 61.50 | 9.25 | 94.10 | 65.30 | 8.25 | 92.80 | 64.40 | 6.0 | 92.60 | 63.70 |
| Comparat ive Formulati on A3 | | | | 8.0 | 96.10 | 69.80 | 7.5 | 91.20 | 63.80 | 3.5 | 80.30 | 59.40 |

The test results in Table 2-1 indicate that when the formulation was added with 5% human serum albumin, the islet score, β-cell expression (efficiency, the proportion of NKX6.1/C-PEP) and islets viability of the formulation liquid decreased significantly within 72H compared to that at 0H. When the formulation was added with 0% and 0.5% human serum albumin, the formulation liquid effectively maintained the islet score, β-cell expression, and islets viability within 72H compared to that at 0H, with no significant difference between the 0% human serum albumin group and the 0.5% human serum albumin group; however, defects of cell aggregates settlement at the bottom of the culture dish or packaging container, more severe adhesion, and significant loss during collection were observed in the 0% human serum albumin group, while defects of adhesion were not observed in the 0.5% human serum albumin group. Furthermore, formulation liquid with 0.10%, 0.25%, 0.625%, 1%, or 2.5% human serum albumin also effectively maintained the islet score, β-cell expression, and islets viability, and no defects of adhesion were observed.

**Table 2-2. Test Results for Different Concentrations of the Stabilizer Histidine**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on A4 | 10 | 97.40 | 61.80 | 8.0 | 98.00 | 61.50 | 7.5 | 98.20 | 60.40 | 7.0 | 97.00 | 58.30 |
| Formulati on A5 | | | | 8.0 | 98.10 | 64.60 | 7.5 | 97.80 | 64.50 | 7.0 | 97.50 | 62.70 |
| Comparat ive Formulati on A6 | | | | 7.0 | 96.90 | 52.70 | 7.0 | 95.90 | 52.00 | 6.0 | 94.80 | 52.40 |

The test results in Table 2-2 indicate that low concentrations of histidine can be used as an auxiliary component of the stabilizer. When low concentration (0-2.0 mg/mL) histidine were added, cell β-cell expression increased accordingly. When high concentration (6.0 mg/mL) histidine were added, there were severe cell adhesion and reductions in both cell viability and β-cell expression.

### Example 2. Formulation Screening for Osmotic Pressure Regulators and pH Regulators

Formulation liquid of islets containing different types and concentrations of osmotic pressure regulators were prepared according to the compositions in Table 4-1, for measuring islet score, cell viability, and the proportion of cell surface markers NKX6.1/C-PEP at 0H, 24H, 48H, and 72H.

The test results indicated that using only physiological saline as the basic solution resulted in poor maintenance of islet aggregates morphology. When using water for injection as the basal medium, sodium chloride, potassium chloride, and calcium chloride are necessary components for maintaining osmotic pressure. If the osmotic pressure is too low, islets exhibit swelling defects.

**Table 4-1. Formulation Screening for Basic Osmotic Pressure Regulator**

| Formulati on No. | Sodium Chloride mg/ml | Potassi um Chlori de mg/ml | Calciu m Chlori de mg/ml | Magne sium Chlori de mg/ml | Glucos e mM | Sodiu m Lactate mg/ml | HSA % | pH | Osmoti c Pressur e |
|---|---|---|---|---|---|---|---|---|---|
| Formula tion B1 | 6.0 | 0.3 | 0.2 | - | - | 3.1 | - | 6.8 | 275 |
| Compar ative Formula tion B2 | - | 0.3 | 0.2 | - | - | 3.1 | - | 6.67 | 76 |
| Compar ative Formula tion B3 | 6.0 | 0.3 | - | - | - | 3.1 | - | 6.71 | 271 |
| Compar ative Formula tion B4 | 6.0 | - | 0.2 | - | - | 3.1 | - | 6.75 | 264 |

**Table 4-2 Test Data for Screening of Basic Osmotic Pressure Regulators**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on B1 | | | | 7.5 | 95.30 | 61.90 | 7.5 | 94.90 | 58.90 | 5.5 | 95.30 | 57.00 |
| Comparat ive Formulati on B2 | | | | 5.0 | 88.30 | 67.40 | 3.0 | 89.70 | 66.20 | 2.0 | 88.70 | 52.50 |
| Comparat ive Formulati on B3 | 10 | 93.40 | 61.70 | 5.5 | 96.80 | 59.30 | 5.5 | 96.70 | 57.80 | 4.5 | 94.70 | 57.30 |
| Comparat ive Formulati on B4 | | | | 7.0 | 97.30 | 61.40 | 7.0 | 95.20 | 56.40 | 5.5 | 94.50 | 55.10 |

Calcium chloride contributed to maintaining the biological activity of islets, and its suitable concentration range was 0.01 mg/ml to 2.0 mg/ml, while at a high concentration of 5.0 mg/ml, the cell aggregates became compact and were difficult to digest.

**Table 5-1. Screening of Calcium Chloride Concentration in Osmotic Pressure Regulators**

| No. | sodium chlorid e mg/ml | Potassi um Chlori de mg/ml | Calciu m Chlori de mg/ml | Magne sium Chlori de mg/ml | Glucos e mM | Sodiu m Lactate mg/ml | HSA % | pH | Osmoti c Pressur e |
|---|---|---|---|---|---|---|---|---|---|
| Comparati ve Formulatio n C1 | 6.0 | 0.3 | 0.01 | - | - | 3.1 | 0.5 | 7.01 | 278 |
| Comparati ve Formulatio n C2 | 6.0 | 0.3 | 5.0 | - | - | 3.1 | 0.5 | 6.8 | 372 |
| Formulatio n B1 | 6.0 | 0.3 | 0.2 | - | - | 3.1 | 0.5 | 6.8 | 275 |
| Formulatio n C3 | 6.0 | 0.3 | 2.0 | | | 3.1 | 0.5 | 6.87 | 319 |

**Table 5-2. Test Data for Screening of Calcium Chloride Concentration in the Osmotic Pressure Regulator**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Comparat ive Formulati on C1 | | | | 6.0 | 97.30 | 58.70 | 4.5 | 95.70 | 58.10 | / | / | / |
| Comparat ive Formulati on C2 | 10 | 94.90 | 56.20 | 7.0 | 93.00 | 65.20 | 6.5 | 69.00 | 63.40 | / | / | / |
| Formulati on B1 | | | | 7.0 | 96.90 | 59.70 | 6.0 | 97.50 | 59.60 | / | / | / |
| Formulati on C3 | | | | 8.0 | 97.00 | 60.60 | 6.5 | 97.20 | 62.20 | / | / | / |

| | | | | | | | | | | | | |
|---|---|---|---|---|---|---|---|---|---|---|---|---|
| Note: '/' indicates that the cell state was abnormal, and data collection for quality parameters was not possible. | | | | | | | | | | | | |

The test results from Tables 6-1 and 6-2 indicate that potassium chloride is a necessary component for maintaining osmotic pressure, with a suitable concentration range of 0-3.0 mg/ml.

**Table 6-1. Screening of Potassium Chloride Concentration in the Osmotic Pressure Regulator**

| No. | Sodiu m Chlori de mg/ml | Potassi um Chlori de mg/ml | Calciu m Chlori de mg/ml | Magne sium Chlori de mg/ml | Glucos e mM | Sodiu m Lactate mg/ml | HSA % | pH | Osmoti c Pressur e |
|---|---|---|---|---|---|---|---|---|---|
| Formulatio n B1 | 6.0 | 0.3 | 0.2 | - | - | 3.1 | 0.5 | 6.8 | 275 |
| Formulatio n D1 | 6.0 | 0 | 0.2 | - | - | 3.1 | 0.5 | 6.75 | 264 |
| Formulatio n D2 | 6.0 | 3.0 | 0.2 | - | | 3.1 | 0.5 | 7.0 | 353 |

**Table 6-2. Test Data for Screening of Potassium Chloride Concentration in the Osmotic Pressure Regulator**

| **Formul ation No** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on B1 | | | | 7 | 96.90 | 59.70 | 6 | 97.50 | 59.60 | / | / | / |
| Formulati on D1 | 10 | 94.90 | 56.20 | 6.5 | 97.60 | 57.90 | 5.5 | 92.70 | 60.10 | / | / | / |
| Formulati on D2 | | | | 6.5 | 96.30 | 58.60 | 5.5 | 92.50 | 60.50 | / | / | / |

The test results from Table 7-1 and Table 7-2 indicate that sodium chloride is a necessary component for maintaining osmotic pressure, and maintains islets morphology by maintaining osmotic pressure. Excessively low concentration of sodium chloride is not conducive to maintaining osmotic pressure, and when the osmotic pressure is relatively low, pancreatic islets exhibit edema defects. Both excessively high and excessively low concentrations of sodium chloride lead to a significant decrease in the islet score at 24H.

**Table 7-1 Screening of Sodium Chloride Concentration in the Osmotic Pressure Regulator**

| No. | Sodiu m Chlori de mg/ml | Potassi um Chlori de mg/ml | Calciu m Chlori de mg/ml | Magne sium Chlori de mg/ml | Glucos e mM | Sodiu m Lactate mg/ml | HSA % | pH | Osmoti c Pressur e |
|---|---|---|---|---|---|---|---|---|---|
| Formulatio n E1 | 1.0 | 0.3 | 0.2 | - | - | 3.1 | 0.5 | 7.06 | 110 |
| Formulatio n B1 | 6.0 | 0.3 | 0.2 | - | - | 3.1 | 0.5 | 6.8 | 275 |
| Formulatio n E2 | 8.0 | 0.3 | 0.2 | - | - | 3.1 | 0.5 | 6.98 | 343 |

**Table 7-2 Test Data for Screening of Sodium Chloride Concentration in the Osmotic Pressure Regulator**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on E1 | | | | 5.5 | 95.90 | 59.70 | 5.5 | 97.40 | 61.90 | / | / | / |
| Formulati on B1 | 10 | 94.90 | 56.20 | 7 | 96.90 | 59.70 | 6 | 97.50 | 59.60 | / | / | / |
| Formulati on E2 | | | | 6.5 | 96.50 | 60.00 | 5.5 | 95.80 | 61.40 | / | / | / |

The test results from Table 8-1 and Table 8-2 indicate that magnesium chloride, as an auxiliary component (advantageous component) of the osmotic pressure regulator, aids in maintaining the proportion of islet β-cells, maintaining cell aggregates morphology, and biological activity.

**Table 8-1 Screening of Magnesium Chloride Concentration in the Osmotic Pressure Regulator**

| No. | Sodiu m Chlori de mg/ml | Potassi um Chlori de mg/ml | Calciu m Chlori de mg/ml | Magne sium Chlori de mg/ml | Glucos e mM | Sodiu m Lactate mg/ml | HAS % | pH | Osmoti c Pressur e |
|---|---|---|---|---|---|---|---|---|---|
| Formulatio n B1 | 6.0 | 0.3 | 0.2 | - | - | 3.1 | 0.5 | 6.8 | 275 |
| Formulatio n F1 | 6.0 | 0.3 | 0.2 | 0.3 | - | 3.1 | 0.5 | 6.81 | 280 |

**Table 8-2 Test Data for Screening of Magnesium Chloride Concentration in the Osmotic Pressure Regulator**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet\ Score** | **Viabi lity %** | **Effici ency %** |
| Formulati on B1 | 10 | 99.2 | 51.2 | 6.5 | 97.20 | 55.40 | 6.0 | 97.40 | 52.30 | 5.5 | 98.10 | 54.00 |
| Formulati on F1 | | | | 7.0 | 97.00 | 55.10 | 6.0 | 97.60 | 55.20 | 6.0 | 96.90 | 55.40 |

The test results from Table 9-1 and Table 9-2 indicate that low concentrations of glucose (0-10 mM) can serve as an auxiliary osmolyte; and the addition of a high glucose concentration (27.7 mM) was observed to cause darkening of the cell aggregates, accompanied by a failure to maintain cell viability and functional markers.

**Table 9-1 Screening of Glucose Concentration in the Osmotic Pressure Regulator**

| No. | Sodium Chlorid e mg/ml | Potassi um Chlorid e mg/ml | Calciu m Chlorid e mg/ml | Magnes ium Chlorid e mg/ml | Glucose mM | Sodium Lactate mg/ml | HAS % | pH | Osmoti c Pressur e |
|---|---|---|---|---|---|---|---|---|---|
| Formulation B1 | 6.0 | 0.3 | 0.2 | - | - | 3.1 | 0.5 | 6.8 | 275 |
| Formulation G1 | 6.0 | 0.3 | 0.2 | - | 10 | 3.1 | 0.5 | 6.94 | 264 |
| Comparative Formulation G2 | 6.0 | 0.3 | 0.2 | - | 27.7 | 3.1 | 0.5 | 6.96 | 296 |

**Table 9-2 Test Data for Screening of Glucose Concentration in the Osmotic Pressure Regulator**

| **Formul ation No** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on B1 | 10 | 97.40 | 51.90 | 7.5 | 95.90 | 52.20 | 7.5 | 96.10 | 52.70 | 6.0 | 95.00 | 52.00 |
| Formulati on G1 | | | | 8.0 | 96.30 | 52.60 | 7.5 | 95.80 | 52.20 | 6.5 | 96.20 | 52.20 |
| Comparat ive Formulati on G2 | | | | 5.5 | 66.40 | 50.10 | 5 | Death | / | 2.5 | Death | / |

The test results from Table 10-1 and Table 10-2 indicate that sodium lactate is a necessary component in the islets formulation liquid, regulating the pH value of the formulation liquid, and well maintains islets biological activity within the concentration range of 1.5 mg/ml to 5.0 mg/ml.

**Table 10-1. Screening of the pH Regulator Sodium Lactate Concentration**

| No. | Sodiu m Chlori de mg/ml | Potassi um Chlori de mg/ml | Calciu m Chlori de mg/ml | Magne sium Chlori de mg/ml | Glucos e mM | Sodiu m Lactate mg/ml | HSA % | pH | Osmoti c Pressur e |
|---|---|---|---|---|---|---|---|---|---|
| Formulatio n H1 | 6.0 | 0.3 | 0.2 | - | - | 0.1 | 0.5 | 6.2 | 213 |
| Formulatio n B1 | 6.0 | 0.3 | 0.2 | - | - | 3.1 | 0.5 | 6.8 | 275 |
| Formulatio n H2 | 6.0 | 0.3 | 0.2 | - | | 1.5 | 0.5 | 6.66 | 243 |
| Formulatio n H3 | 6.0 | 0.3 | 0.2 | - | | 5.0 | 0.5 | 7.05 | 316 |

**Table 10-2 Test Data for Screening of the pH Regulator Sodium Lactate Concentration**

| **Formul ation No.** | **0H** | | | **24H** | | | **48H** | | | **72H** | | |
|---|---|---|---|---|---|---|---|---|---|---|---|---|
| | **Islet Score** | **Viabi lity %** | **Effici ency %** | **Islet Score** | **Viabi lity %** | **Effici ency %** | **Islet Score** | **Viabi lity %** | **Effici ency %** | **Islet Score** | **Viabi lity %** | **Effici ency %** |
| Formulati on H1 | 10 | 94.90 | 56.20 | 5.5 | 96.80 | 60.70 | 4.5 | 91.80 | 59.70 | / | / | / |
| Formulati on B1 | | | | 7 | 96.90 | 59.70 | 6 | 97.50 | 59.60 | / | / | / |
| Formulati on H2 | | | | 6.5 | 97.40 | 61.80 | 5.5 | 93.00 | 62.50 | / | / | / |
| Formulati on H3 | | | | 6.5 | 97.60 | 61.60 | 5 | 94.20 | 63.20 | / | / | / |

### Example 3. Screening of Cell Density in the Formulation

Islets formulation liquids with cell densities of 1,000 IEQ/mL, 6,000 IEQ/mL, and 9,000 IEQ/mL were prepared using Formulation B1 from Example 2, for measuring islet score, cell viability (%), and the proportion of cell surface markers NKX6.1/C-PEP (efficiency %) at 0H, 24H, 48H, and 72H.

The test results indicate that, at a higher formulation density (9,000 IEQ/mL), the viability of the islets, β-cell expression, and islet score all exhibited a marked decrease over 72 hours of storage, accompanied by noticeably worsened cell adhesion. These drawbacks were mitigated upon reduction of the formulation density (below 8,000 IEQ/mL).

**Table 10 Test Data for Cell Density Screening**

| **Cell Density /mL** | **0H** | | | **24H** | | | **48H** | | | **72H** | | |
|---|---|---|---|---|---|---|---|---|---|---|---|---|
| | **Islet Scor e** | **Viab ility %** | **Effic iency %** | **Islet Scor e** | **Viab ility %** | **Effic iency %** | **Islet Scor e** | **Viab ility %** | **Effic iency %** | **Islet Scor e** | **Viab ility %** | **Effic ienc y %** |
| 1000 IEQ | 10 | 94.60 | 70.90 | 9 | 94.40 | 74.00 | 8.5 | 94.70 | 69.90 | 7.5 | 91.20 | 72.70 |
| 6000 IEQ | | | | 9 | 94.20 | 71.40 | 8.5 | 93.40 | 70.40 | 7 | 94.30 | 74.80 |
| 9000 IEQ | | | | 8.5 | 94.40 | 74.70 | 8 | 91.60 | 75.20 | 6.5 | 89.90 | 69.10 |

### Example 4. Screening of Storage Temperature for the Formulation Liquid

Islets formulation liquids were prepared using Formulation B1 from Example 2, for measuring islet score, cell viability (%), and the proportion of cell surface markers NKX6.1/C-PEP (efficiency %) at 0H, 24H, 48H, and 72H under conditions of 4°C, 22°C, and 37°C, respectively. The test results indicate that the example formulation liquid was capable of maintaining the islet score, cell viability, and NKX6.1/C-PEP level under conditions of 4°C-22°C, which is beneficial for the temperature-controlled transportation of the formulation.

**Table 11. Test Data for Storage Temperature**

| **Tempe rature** | **0H** | | | **24H** | | | **48H** | | | **72H** | | |
|---|---|---|---|---|---|---|---|---|---|---|---|---|
| | **Islet Scor e** | **Viab ility %** | **Effic iency %** | **Islet Scor e** | **Viab ility %** | **Effic iency %** | **Islet Scor e** | **Viab ility %** | **Effic iency %** | **Islet Scor e** | **Viab ility %** | **Effic ienc y %** |
| 4°C | 10 | 91.2 | 62.1 | 9.0 | 95.50 | 59.40 | 8.5 | 91.30 | 59.70 | \ | \ | \ |
| 22 °C | | | | 9.0 | 95.00 | 64.70 | 8.0 | 93.90 | 62.50 | \ | \ | \ |
| 37°C | | | | 3.5 | 63.70 | 41.10 | 1.5 | 44.20 | 24.70 | \ | \ | \ |

### Example 5. Cell Viability and Islets Proportions in Different Cell Formulation Liquids

Human pluripotent stem cell-derived islet aggregates were taken, the culture supernatant was discarded, and the formulation liquid was added. At time points of 0H, 24H, 48H, and 72H after formulation, an equal amount of cell aggregates was taken out. A single cell suspension was obtained by digesting with Accutase enzyme solution for 15-25 minutes. Samples were taken to detect cell viability using the NC200 cell counter. The expression of functional markers was detected by flow cytometry. The proportion of islet β cells was determined by the double-positive proportion for NKX6.1 and C-PEP. The proportion of islet α cells was determined by the double-positive proportion for ARX and GCG. The proportion of islet δ cells was determined by the double-positive proportion for SST and HHEX. The proportion of endocrine cells was determined by the CHGA-positive proportion. Experimental procedures for the daily sampling method, sample volume, digestion method, and final measurements were kept consistent.

**Table 12. Formulations of Compound Formulation Liquids**

| **No.** | **Name of the Formulation Liquid** | **Formulation Components** |
|---|---|---|
| 1 | Compound Formulation Liquid 1 | It contains 0.02 g Calcium Chloride, 0.03 g Potassium Chloride, 0.6 g Sodium Chloride, 0.31 g Sodium Lactate, 0.03 g Magnesium Chloride, 0.2 g Histidine, and 0.5% HSA per 100 mL. |
| 2 | Compound Formulation Liquid 2 | It contains 0.9% Sodium Chloride and 0.5% HAS. |
| 3 | Compound Formulation Liquid 3 | It contains 2.92 g Sodium Chloride, 0.15 g Potassium Chloride, 0.11 g Calcium Chloride, 0.1 g Magnesium Chloride, 1.175 g Sodium Bicarbonate, 0.1 g Sodium Citrate, an appropriate amount (about 0.07 g) of Citric Acid, and 0.5% HSA per 500 mL. |
| 4 | FRS | It contains s sugars, salts, pH buffer, energy substrates, osmotic stabilizers, free radical scavenging components, and cryoprotectants (including DMSO) |

The test results in Table 13 indicate that at 24H after formulation, the cell viability in the Compound Formulation Liquid 1, Compound Formulation Liquid 2, and Compound Formulation Liquid 3 groups was relatively high, above 90%; at 48H after formulation, when using Compound Formulation Liquid 1 and Compound Formulation Liquid 2, the cell viability remained stable, still maintained above 90%, while the cell viability in the Compound Formulation Liquid 3 group decreased significantly; and at 72H after formulation, the cell viability in the Compound Formulation Liquid 1 group was the highest, remaining stable above 90%.

**Table 13. Test Results of Islets Viability**

| **Name of the Formulation Liquid** | **0H** | **24H** | **48H** | **72H** |
|---|---|---|---|---|
| Compound Formulation Liquid 1 | 96.1% | 95.1% | 92.9% | 91.6% |
| Compound Formulation Liquid 2 | 96.1% | 91.4% | 90.1% | 88.0% |
| Compound Formulation Liquid 3 | 96.1% | 94.4% | 85.4% | 68.2% |
| FRS | 96.1% | 86.3% | 78.7% | 75.5% |

The test results from Table 14 indicate that within 72 hours after formulation, the expression proportion of islet α cells remained relatively stable across all groups, with no significant differences.

**Table 14. Comparison of Islet α Cell Proportion Across Different Formulation Liquid Types**

| **Name of the Formulation Liquid** | **0H** | **24H** | **48H** | **72H** |
|---|---|---|---|---|
| Compound Formulation Liquid 1 | 21.6% | 24.8% | 25.2% | 25.5% |
| Compound Formulation Liquid 2 | 21.6% | 26.7% | 27.2% | 27.0% |
| Compound Formulation Liquid 3 | 21.6% | 24.0% | 24.0% | 23.9% |
| FRS | 21.6% | 26.1% | 25.5% | 25.8% |

The test results from Table 15 indicate that within 24 hours after formulation, the expression proportion of islet β cells in all other groups was above 40%, with the Compound Formulation Liquid 1 group being the highest. Within 72 hours after formulation, the Compound Formulation Liquid 1 group was significantly superior to the other groups in maintaining the proportion of islet β cells.

**Table 15. Comparison of Islet β Cell Proportion Across Different Formulation Liquid Types**

| **Name of the Formulation Liquid** | **0H** | **24H** | **48H** | **72H** |
|---|---|---|---|---|
| Compound Formulation Liquid 1 | 42.4% | 45.6% | 46.4% | 41.3% |
| Compound Formulation Liquid 2 | 42.4% | 42.5% | 40.9% | 31.6% |
| Compound Formulation Liquid 3 | 42.4% | 41.8% | 39.5% | 32.5% |
| FRS | 42.4% | 41.5% | 40.7% | 35.6% |

The test results from Table 16 indicate that within 24 hours after formulation, the difference in the expression proportion of islet δ cells among the groups was small, with the Compound Formulation Liquid 1 group and the Compound Formulation Liquid 2 group showing relatively higher proportions. At 48H-72H of formulation, the expression proportion of islet δ cells in the Compound Formulation Liquid 3 group and the FRS group decreased significantly; and the expression of islet δ cells in the Compound Formulation Liquid 1 group and the Compound Formulation Liquid 2 group remained relatively stable.

**Table 16. Comparison of Islet δ Cell Proportion Across Different Formulation Liquid Types**

| **Name of the Formulation Liquid** | **0H** | **24H** | **48H** | **72H** |
|---|---|---|---|---|
| Compound Formulation Liquid 1 | 8.18% | 10.10% | 9.80% | 9.21% |
| Compound Formulation Liquid 2 | 8.18% | 10.40% | 9.50% | 9.10% |
| Compound Formulation Liquid 3 | 8.18% | 8.50% | 3.70% | 3.60% |
| FRS | 8.18% | 7.50% | 5.00% | 3.90% |

The test results from Table 17 indicate that within 24 hours after formulation, there was no significant difference in the expression proportion of islet endocrine cells among the groups; within 72 hours after formulation, the expression of endocrine cells in the Compound Formulation Liquid 1 group and the Compound Formulation Liquid 2 group was relatively stable and was significantly superior to the other islets formulation liquid groups.

**Table 17. Statistical Table of Comparison of Endocrine Cell Proportion Across Different Formulation Liquid Types**

| **Name of the Formulation Liquid** | **0H** | **24H** | **48H** | **72H** |
|---|---|---|---|---|
| Compound Formulation Liquid 1 | 85.1% | 88.8% | 89.9% | 89.1% |
| Compound Formulation Liquid 2 | 85.1% | 88.3% | 88.3% | 88.5% |
| Compound Formulation Liquid 3 | 85.1% | 80.5% | 80.6% | 81.9% |
| FRS | 85.1% | 87.3% | 86.1% | 85.5% |

## Claims

1. A formulation of human pluripotent stem cell-derived islets, comprising pluripotent stem cell (hPSC)-derived islets in the form of cell aggregates with a diameter greater than 50 µm, 0%-5% human serum albumin, and a basal medium.

2. The formulation of human pluripotent stem cell-derived islets according to claim 1, wherein the pluripotent stem cell (hPSC)-derived islet aggregates in per milliliter formulation equivalent to 1-20 million single cells derived from human pluripotent stem cells; or the density of pluripotent stem cell (hPSC)-derived islet aggregates in per milliliter formulation is 1,000-10,000 IEQ;
preferably, wherein the pluripotent stem cell (hPSC)-derived islet aggregates in per milliliter formulation equivalent to 2-16 million single cells derived from human pluripotent stem cells; or the density of pluripotent stem cell (hPSC)-derived islet aggregates in per milliliter formulation is 2,000-8,000 IEQ.

3. The formulation of human pluripotent stem cell-derived islets according to claim 1 or 2, further comprises an osmotic pressure regulator and a pH regulator.

4. The formulation of human pluripotent stem cell-derived islets according to claim 3, wherein the osmotic pressure regulator is selected from the group consisting of:
sodium chloride, potassium chloride, calcium chloride, magnesium chloride, zinc sulfate, glucose, trehalose, or a combination thereof;
preferably, a combination of sodium chloride, potassium chloride and calcium chloride; or a combination of sodium chloride, potassium chloride, calcium chloride and magnesium chloride, or a combination of sodium chloride, potassium chloride, calcium chloride and glucose.

5. The formulation of human pluripotent stem cell-derived islets according to claim 3, wherein the pH regulator controls the pH of the formulation in the range of 6.5-8.0;
the pH regulator is selected from the group consisting of sodium lactate, sodium acetate, HEPES, sodium bicarbonate, sodium citrate, citric acid, or a combination thereof; preferably sodium lactate, HEPES, or a combination of sodium bicarbonate, sodium citrate and citric acid.

6. The formulation of human pluripotent stem cell-derived islets according to any one of claims 1-5, further comprises an antioxidant or an additional stabilizer, or a combination thereof.

7. The formulation of human pluripotent stem cell-derived islets according to claim 6, wherein the additional stabilizer is selected from one or more of histidine, sodium gluconate, pyruvate, Nicotinamide ITS (1X), linoleic acid, and Vitamin E.

8. The formulation of human pluripotent stem cell-derived islets according to claim 1, comprises:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 1-20 million single cells derived from human pluripotent stem cells; or the density of the pluripotent stem cell (hPSC)-derived islet aggregates in per milliliter formulation is 1,000-10,000 IEQ;
0%-5% human serum albumin;
an osmotic pressure regulator, which is selected from a combination of sodium chloride, potassium chloride, and calcium chloride, or the combination with further addition of magnesium chloride;
a pH regulator, which is selected from sodium lactate, or a combination of sodium bicarbonate, sodium citrate and citric acid;
optionally, an additional stabilizer, which is selected from histidine, sodium gluconate, or a combination thereof; and
optionally, an antioxidant, which is selected from one or more of pyruvate and Nicotinamide.

9. The formulation of human pluripotent stem cell-derived islets according to claim 1, comprises:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 2-16 million single cells derived from human pluripotent stem cells; or the density of the pluripotent stem cell (hPSC)-derived islet aggregates in per milliliter formulation is 2,000-8,000 IEQ;
0%-5% human serum albumin;
an osmotic pressure regulator, which is selected from the group consisting of 1.0-9.0 mg/ml sodium chloride, 0.1-3.0 mg/ml potassium chloride, 0.01-2.0 mg/ml calcium chloride, and 0-1.0 mg/ml magnesium chloride;
preferably a combination of 6.0 mg/ml sodium chloride, 0.3 mg/ml potassium chloride and 0.2 mg/ml calcium chloride; or a combination of 6.0 mg/ml sodium chloride, 0.3 mg/ml potassium chloride, 0.2 mg/ml calcium chloride and 0.3 mg/ml magnesium chloride;
a pH regulator, which is selected from 1.5-5.0 mg/ml sodium lactate, 20-30 mM HEPES, or a combination of 2.35 mg/ml sodium bicarbonate, 0.2 mg/ml sodium citrate and 0.14 mg/ml citric acid; preferably 3.1 mg/ml sodium lactate, 25 mM HEPES, or a combination of 2.35 mg/ml sodium bicarbonate, 0.2 mg/ml sodium citrate and 0.14 mg/ml citric acid;
optionally, an additional stabilizer, which is selected from 0-6.0 mg/ml histidine; and
optionally, an antioxidant, which is selected from 0-10 mM Nicotinamide.

10. The formulation of human pluripotent stem cell-derived islets according to claim 1, comprises:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 2-16 million single cells derived from human pluripotent stem cells; or the density of the pluripotent stem cell (hPSC)-derived islet aggregates in per milliliter formulation is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
3.1 mg/ml sodium lactate;
0-6.0 mg/ml histidine, preferably 0-2.0 mg/ml; and
0-10 mM Nicotinamide.

11. The formulation of human pluripotent stem cell-derived islets according to claim 1, comprises:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 2-16 million single cells derived from human pluripotent stem cells; or the density of the pluripotent stem cell (hPSC)-derived islet aggregates in per milliliter formulation is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
25 mM HEPES or a combination of 25 mM HEPES and 3.1 mg/ml sodium lactate;
0-2.0 mg/ml histidine; and
0-10 mM Nicotinamide.

12. The formulation of human pluripotent stem cell-derived islets according to claim 1, comprises:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 2-16 million single cells derived from human pluripotent stem cells; or the density of the pluripotent stem cell (hPSC)-derived islet aggregates in per milliliter formulation is 2,000-8,000 IEQ;
0%-5% human serum albumin;
a combination of 6.0 mg/ml sodium chloride, 0.3 mg/ml potassium chloride, 0.2 mg/ml calcium chloride and 0.3 mg/ml magnesium chloride;
a combination of 2.35 mg/ml sodium bicarbonate, 0.2 mg/ml sodium citrate and 0.14 mg/ml citric acid;
optionally, an additional stabilizer, which is selected from 0-2.0 mg/ml histidine;
optionally, an antioxidant, which is selected from 0-10 mM Nicotinamide.

13. The human formulation of pluripotent stem cell-derived islets according to claim 1, comprising:
the pluripotent stem cell (hPSC)-derived islets in the form of cell aggregates with a diameter of 50-350 µm, wherein the pluripotent stem cell (hPSC)-derived islet aggregates in per milliliter formulation equivalent to 2-16 million single cells derived from human pluripotent stem cells; or the density of the pluripotent stem cell (hPSC)-derived islet aggregates in per milliliter formulation is 2,000-8,000 IEQ;
0%-5% human serum albumin;
9.0 mg/ml sodium chloride (equivalent to 0.9% physiological saline); optionally, an additional stabilizer, which is selected from 0-2.0 mg/ml histidine;
optionally, an antioxidant, which is selected from 0-10 mM Nicotinamide.

14. The formulation of human pluripotent stem cell-derived islets according to any one of claims 1 to 13, wherein the human pluripotent stem cell-derived islets are NKX6.1+ and C-PEP+ double-positive;
preferably, after the formulation is stored at 4°C to 28°C (±3°C) for 24 hours, at least 20% of human pluripotent stem cell-derived islets are NKX6.1+ and C-PEP+ double-positive; preferably, at least 30% of human pluripotent stem cell-derived islets are NKX6.1+ and C-PEP+ double-positive, at least 35% of human pluripotent stem cell-derived islets are NKX6.1+ and C-PEP+ double-positive, at least 40% of human pluripotent stem cell-derived islets are NKX6.1+ and C-PEP+ double-positive, at least 45% of human pluripotent stem cell-derived islets are NKX6.1+ and C-PEP+ double-positive, at least 50% of human pluripotent stem cell-derived islets are NKX6.1+ and C-PEP+ double-positive, at least 55% of human pluripotent stem cell-derived islets are NKX6.1+ and C-PEP+ double-positive.

15. The formulation of human pluripotent stem cell-derived islets according to any one of claims 1 to 14, wherein the pluripotent stem cell-derived islets are cell aggregates with a diameter of 50-350 µm.

16. The formulation of human pluripotent stem cell-derived islets according to any one of claims 1 to 7, after storing at 4°C to 28°C (±3°C) for 24 hours, preferably at 4°C to 24°C (±3°C) for 24 hours, the human pluripotent stem cell-derived islets maintain cell viability (biological activity) above 85%, preferably maintain biological activity above 90%, 92.5%, or 95%.

17. The formulation of human pluripotent stem cell-derived islets according to any one of claims 1 to 16, wherein the formulation is an injection, preferably, the formulation is an injection administrated in hepatic portal vein or under rectus sheath, more preferably, under anterior rectus sheath.

18. A process for preparing the formulation of human pluripotent stem cell-derived islets according to any one of claims 1-17, comprises the steps of:
(1) preparing the cell preservation composition formulation: adding human serum albumin and other components to the basal medium, and mixing uniformly to prepare a cell preservation composition for human pluripotent stem cell-derived islets; and
(2) mixing the cell preservation composition with human pluripotent stem cell-derived islets, and filling into a packaging container.

19. The formulation of human pluripotent stem cell-derived islets according to any one of claims 1-17 for use for the treatment of insulin deficiency or the treatment of an insulin deficiency disease;
preferably, the formulation is an injection administrated in hepatic portal vein or under rectus sheath, more preferably, the formulation is administrated under anterior rectus sheath.

20. Use of a cell preservation composition for human pluripotent stem cell-derived islets in long-distance transportation, wherein the preservation composition consists of the components in the formulation according to any one of claims 1-17 without human pluripotent stem cell-derived islets, and the long-distance transportation temperature is 4°C to 37°C; and the transportation time does not exceed 96 hours;
preferably, the long-distance transportation temperature is 4°C, 10°C, 16°C, 22°C, or 28°C; and
preferably, the transportation time does not exceed 96 hours, 72 hours, 48 hours, 36 hours, 24 hours, 20 hours, 16 hours, 12 hours, 8 hours, 6 hours, or 4 hours.
